Europäisches Patentamt

European Patent Office

Office européen des brevets

(19)

(11) Veröffentlichungsnummer: **0 544 292 A2**

# (12) EUROPÄISCHE PATENTANMELDUNG

(21) Anmeldenummer: **92120205.7**

(22) Anmeldetag: **26.11.92**

(51) Int. Cl.5: **C12N 15/87**, C12N 15/62

(30) Priorität: **27.11.91 DE 4139001**

(43) Veröffentlichungstag der Anmeldung:
**02.06.93 Patentblatt 93/22**

(84) Benannte Vertragsstaaten:
**AT BE CH DE DK ES FR GB GR IE IT LI LU NL PT SE**

(71) Anmelder: **BOEHRINGER MANNHEIM GMBH Sandhofer Strasse 112-132 W-6800 Mannheim-Waldhof(DE)**

(72) Erfinder: **Stern, Anne, Dr.**
**Karwendelstrasse 10**
**W-8122 Penzberg(DE)**
Erfinder: **Hagemann, Irene**
**Eigerstrasse 46**
**W-8000 München 82(DE)**
Erfinder: **Ziegler-Landesberger, Doris**
**Einsteinstrasse 7**
**W-8033 Planegg(DE)**

(74) Vertreter: **Huber, Bernhard, Dipl.-Chem. et al Patentanwälte H. Weickmann, Dr. K. Fincke F.A. Weickmann, B. Huber Dr. H. Liska, Dr. J. Prechtel, Dr. B. Böhm, Kopernikusstrasse 9, Postfach 86 08 20 W-8000 München 86 (DE)**

(54) **Verfahren zur Einschleusung von Nukleinsäuren in Zellen.**

(57) Die vorliegende Erfindung betrifft ein Verfahren zur Herstellung eines Komplexes für die Einschleusung von Nukleinsäuren in Zielzellen durch nichtkonvalente Komplexbildung einer Nukleinsäure mit einem Protein, das eine für die Zielzelle spezifische "Cell Homing Faktor"-Komponente und mindestens eine Polykation-Komponente enthält, wobei ein Protein, das eine lineare genetische Fusion aus der "Cell Homing Faktor"- und mindestens einer Polykation-Komponente enthält, mit der in die Zielzellen einzuschleusenden Nukleinsäure unter geeigneten Bedingungen in Kontakt gebracht wird, so daß ein im wesentlichen auf ionischen Wechselwirkungen zwischen der Nukleinsäure und dem Polykation-Anteil des Proteins beruhender Komplex gebildet wird, wobei eine Polykation-Komponente des Proteins eine Peptidsequenz ist, die mindestens 3 Aminosäuren, ausgewählt aus Lysin und Arginin, enthält und eine unter physiologischen Bedingungen stabile Bindung mit Nukleinsäuren bilden kann.

EP 0 544 292 A2

Die vorliegende Erfindung betrifft ein Verfahren zur Herstellung eines Komplexes für die Einschleusung von Nukleinsäuren in Zielzellen durch nicht-kovalente Komplexbildung einer Nukleinsäure mit einem Protein, das eine für die Zielzelle spezifische Komponente und eine Polykation-Komponente enthält. Weiterhin betrifft die vorliegende Erfindung ein Verfahren zur Einschleusung derartiger Komplexe in Zielzellen.

In den vergangenen Jahren wurden zahlreiche Methoden entwickelt, um Nukleinsäuren in Zellen, insbesondere eukaryontische Zellen einzuführen. Beispiele für derartige Methoden sind etwa Calciumphosphat-Transfektion, Polybren-Transfektion, Protoplastenfusion, Elektroporation, Lipofektion und Mikroinjektion (siehe z.B. Sambrook et al., (1989) Molecular Cloning, A Laboratory Manual, Cold Spring Harbor). Die oben genannten Methoden finden in vitro eine breite Anwendung, das Potential der Einschleusung von Nukleinsäuren in vivo nach diesen Methoden ist jedoch aus verschiedenen Gründen, wie z.B. fehlende Zellspezifität, Ausbildung unlöslicher Präzipitate, geringe Effizienz und Machbarkeit und dgl. stark limitiert.

Daher hat man in der jüngsten Zeit an der Entwicklung von sogenannten "Nukleinsäure-Delivery-Systemen" gearbeitet, die auf der Idee beruhen, Nukleinsäuremoleküle ohne Beeinträchtigung der Zielzelle effizient und spezifisch in die Zellen einzuschleusen. Hintergrund dieser Anstrengungen bilden die Bemühungen um Nukleinsäuretherapeutika, mit deren Hilfe man zusätzliche genetische Informationen in die Zellen eines bestimmten Gewebes einschleusen oder die Expression eines bestimmten Gens in einem spezifischen Gewebe durch Einschleusung von Antisense-Molekülen oder Ribozymen unterdrücken will.

Zu diesem Zweck wurde versucht, die Fähigkeit von Nukleinsäuren zur Durchdringung von Membranen zu verbessern. Dies gelang beispielsweise dadurch, daß man die Nukleinsäure kovalent mit Polykationen wie Polylysin (Lemaitre et al., Proc.Natl.Acad.Sci. USA 84 (1987), 648) oder mit amphiphilen Molekülen wie etwa Polyethylenglykol (WO 88/09810) koppelte. Damit konnte zwar die Effizienz der Einschleusung von Nukleinsäuren in die Zelle verbessert werden, das Problem der Zellspezifität bestand jedoch noch immer.

Zur Erzeugung eines zellspezifischen Nukleinsäure-Delivery-Systems wurde vorgeschlagen, die in die Zelle einzuschleusende Nukleinsäure direkt an einen sogenannten "Cell Homing Faktor", d.h. einen Faktor, der spezifische Zellmarker erkennt bzw. eine Affinität für spezifische Zellrezeptoren aufweist, wie etwa Epidermal Growth Factor (EGF) konjugiert (EP-A 0 273 085). Ein Nachteil dieses Verfahrens ist jedoch, daß die Nukleinsäure in die Zelle nicht in freier Form, sondern in Form eines kovalenten Konjugats eingeschleust wird.

Eine weitere Methode zur Einschleusung von Nukleinsäuren in Zielzellen besteht darin, die Nukleinsäuren nicht-kovalent mit Konjugaten aus Proteinen und Polykationen zu koppeln. Arbeiten von Wu und Wu (J.Biol.Chem. (1987), 4429-4432; J.Biol.Chem. 263 (1988), 14621-14624) offenbaren die Einschleusung von fremder DNA in Zellen mit Hilfe eines löslichen DNA-Trägersystems, das aus einem chemisch synthetisierten kovalenten Konjugat mit einem Glycoprotein besteht. Die EP-A 0 388 758 offenbart chemisch synthetisierte TransferrinPolykation-Konjugate,die mit polyanionischen Nukleinsäuren Komplexe bilden, die durch Bindung an den Transferrinrezeptor in Zielzellen eingeschleust werden können.

Ein Nachteil dieser oben genannte Systeme besteht jedoch darin, daß bei der chemischen Herstellung von Konjugaten zumeist sehr heterogene Produkte entstehen, d.h. daß ein Konjugat sehr unterschiedliche Anteile an den jeweiligen Einzelkomponenten enthalten kann. Des weiteren besteht die Gefahr, daß in einem Teil der produzierten Konjugate ein Teil der assoziierten Proteinkomponenten in ihrer Funktionalität (d.h. in ihrer Fähigkeit zum Eindringen in eine Zielzelle) beeinträchtigt sind. Dies wiederum ist für die Entwicklung eines therapeutischen Produkts sehr ungünstig.

Aufgabe der vorliegenden Erfindung war es somit, ein Verfahren zur Bereitstellung eines zellspezifischen Nukleinsäure-Delivery-Systems zu entwickeln, bei dem die Nachteile des Standes der Technik zumindestens teilweise beseitigt sind.

Die erfindungsgemäße Aufgabe wird durch ein Verfahren zur Herstellung eines Komplexes für die Einschleusung von Nukleinsäuren in Zielzellen gelöst, wobei eine nicht-kovalente Komplexbildung einer Nukleinsäure mit einem Protein, das eine für die Zielzelle spezifische "Cell Homing Faktor"-Komponente und mindestens eine Polykation-Komponente enthält, welches dadurch gekennzeichnet ist, daß man ein Protein, das eine lineare genetische Fusion aus der "Cell Homing Faktor"- und mindestens einer Polykation-Komponente enthält, mit der in die Zielzellen einzuschleusenden Nukleinsäure unter geeigneten Bedingungen in Kontakt bringt, so daß ein im wesentlichen auf ionischen Wechselwirkungen zwischen der Nukleinsäure und dem Polykation-Anteil des Proteins beruhender Komplex gebildet wird, wobei eine Polykation-Komponente des Proteins eine Peptidsequenz ist, die mindestens 3 Aminosäuren, ausgewählt aus Lysin und Arginin, enthält und eine unter physiologischen Bedingungen stabile Bindung mit Nukleinsäuren bilden kann.

Die Nukleinsäure, die einen Komplex mit dem Protein bildet, kann eine lineare oder zirkuläre, einzel- oder doppelsträngige DNA oder RNA oder ein DNA-RNA-Hybrid sein. Ferner kann die Nukleinsäure auch chemisch modifiziert sein, sofern die negative Ladung des Phosphatrückgrats erhalten bleibt, welche die ionische Bindung an die Polykation-Komponente des Proteins vermittelt. Beispiele für geeignete modifizierte Nukleinsäuren stellen etwa die Thioate und Dithioate dar. In diesem Zusammenhang ist auf den Review-Artikel von Uhlmann und Peyman (1990), Chemical Reviews 90 (4), 544-584 zu verweisen, in dem auch noch weitere geeignete Nukleinsäurederivate aufgezählt sind.

Ferner können auch Nukleinsäuren mit chemisch modifizierten Nukleotidbasen, z.B. RNA-Moleküle, bei denen die 2'-OH-Gruppe in einem oder mehreren Nukleotiden durch eine O-Alkylgruppe, Halogengruppe oder andere Modifizierungsgruppen ersetzt ist, verwendet werden.

Vorzugsweise ist die in die Zielzellen einzuschleusende Nukleinsäure eine DNA oder eine gegebenenfalls modifizierte RNA. Die in die Zielzelle eingeschleuste Nukleinsäure kann etwa genetische Informationen enthalten, die in der Zielzelle exprimiert werden. Somit ist beispielsweise eine Beseitigung genetisch bedingter Defekte möglich. Andererseits kann die in die Zielzelle einzuschleusende Nukleinsäure auch Antisense-Eigenschaften (d.h. die Nukleinsäure ist komlementär zu einer der Zielzelle vorliegenden mRNA) zur Hemmung der Expression spezifischer Gene in der Zielzelle besitzen. Schließlich kann die einzuschleusende Nukleinsäure auch Ribozymeigenschaften besitzen, d.h. sie ist zur Spaltung von spezifischen RNA-Molekülen in der Zielzelle in der Lage. Beispiele für derartige Ribozyme sind etwa die Hammerhead-Ribozyme, die in Rossi und Sarver, Tibtech 8 (1990) 179 - 183) beschrieben sind. Die Einführung von Antisense- oder Ribozymnukleinsäuren in spezifische Zielzellen kann insbesondere bei der Therapie viraler Erkrankungen, z.B. AIDS, eine wichtige Rolle spielen.

Das für das erfindungsgemäße Verfahren verwendete Protein ist eine lineare genetische Fusion aus einer "Cell Homing Faktor"- und einer oder mehrerer Polykation-Komponenten.

Das "Protein" im Sinne der vorliegenden Erfindung kann zusätzlich zu seinem Proteinanteil noch Zucker-, Lipid- oder/und Phosphatreste enthalten. Die Aufgabe der Cell Homing Faktor-Komponente besteht darin, die Nukleinsäure in das Innere der Zielzelle, vorzugsweise durch rezeptorvermittelte Endozytose in das Innere der Zielzelle zu transportieren. Bei einer Cell Homing Faktor-Komponente im Sinne der vorliegenden Erfindung handelt es sich um einen Proteinfaktor, der Zellmarker-erkennende Eigenschaften besitzt, bzw. eine spezifische Affinität für einen Zellrezeptor aufweist. Bevorzugt werden Faktoren, die nach Wechselwirkung mit dem Zellmarker bzw. Rezeptor internalisiert werden. Vorzugsweise wird die Cell Homing Faktor-Komponente aus der Gruppe, bestehend aus Wachstumsfaktoren, Hormonen, viralen Antigenen, Toxinen, Lipoproteinen und Integrinen ausgewählt. Besonders bevorzugt verwendet man als Cell Homing Faktor-Komponente einen Wachstumsfaktor, insbesondere G-CSF (Granulozyten-Kolonien-stimulierender Faktor) oder NGF (Nerve Growth Factor).

Weitere Beispiele für Cell Homing Faktor-Komponenten, die für das erfindungsgemäße Verfahren geeignet sind, sind andere Wachstumsfaktoren, wie z.B. PDGF (Platelet Derived Growth Factor) und EGF (Epidermal Growth Factor), die mit dem jeweiligen Wachstumsfaktor-Rezeptor (PDGF-R bzw. EGF-R) in Wechselwirkung treten. Ein weiteres Beispiel ist die Wechselwirkung von viralen Antigenen mit den entsprechenden Rezeptoren (z.B. gp124 von HIV mit dem CD4-Oberflächenmarker auf T-Helferzellen), die Wechselwirkung von Toxinen mit ihren Rezeptoren ($\beta$-Anteil von Rizin, Diphtherietoxin), die Wechselwirkung von LDL mit dem LDL-Rezeptor, die Wechselwirkung von Hormonen mit ihren Rezeptoren (z.B. Insulin und Insulin-Rezeptor) sowie die Wechselwirkung von Integrinen mit ihren Rezeptoren (z.B. Vironektin und Plättchenfaktor gp IIIA/IIB).

Die Polykation-Komponente des Proteins für das erfindungsgemäße Verfahren ist eine Peptidsequenz, die mindestens drei Aminosäuren, ausgewählt aus Lysin und Arginin, enthält und eine unter physiologischen Bedingungen stabile Bindung mit Nukleinsäuren bildet. Neben den positiv geladenen Aminosäuren Lysin und Arginin kann die Polykation-Komponente auch neutrale Aminosäuren enthalten, solange ihr Gesamtcharakter immer noch ausreichend kationisch bleibt, um eine unter physiologischen Bedingungen stabile Bindung an Nukleinsäuren zu bilden. Dies läßt sich durch einfache Versuche beim Zusetzen von Nukleinsäuren überprüfen. Insbesondere sind solche Proteine für das erfindungsgemäße Verfahren geeignet, die nach Inkubation bei Raumtemperatur in 25 mmol/l NaCl, einem pH zwischen 7 und 8 und in Gegenwart einer Nukleinsäurekonzentration von 25 ng/$\mu$l bei einer Proteinkonzentration von 0,01 - 1 mg/ml einen Protein-Nukleinsäure-Komplex mit ausreichender Stabilität bilden, um bei anschließender Agarose-Gelelektrophorese eine Retardation der Nukleinsäurebanden zu bewirken. Diese Retardation der Nukleinsäurebanden zeigt, daß der Komplex aus Konjugat und Nukleinsäure während der Agarose-Gelelektrophorese erhalten bleibt.

Die Polykation-Komponente des Proteins muß mindestens 3 Lysin- oder Arginin-Reste enthalten, während sich eine Obergrenze nicht angeben läßt. So ist beispielsweise aus der Literaturstelle E. Wagner

EP 0 544 292 A2

(Proc.Natl.Acad.Sci. USA 87 (1990) 3410-3414) bekannt, daß Polykation-Komponenten bis zu 450 Aminosäuren enthalten können und immer noch funktionieren. Vorzugsweise beträgt die Länge der Polykation-Komponente jedoch 5 bis 100 Aminosäuren. Der Anteil der kationischen Aminosäuren in der Kette der Polykation-Komponente sollte vorzugsweise gleich oder größer 40 mol-%, besonders bevorzugt mindestens 60 mol-% sein, obwohl auch Polykation-Komponenten mit einem geringeren Anteil an Arg- oder/und Lys-Resten eine stabile Bindung mit Nukleinsäuren bilden können.

Besonders bevorzugt ist, wenn die Polykation-Komponente des Proteins mindestens 4 Arg- oder/und Lys-Reste und bis zu 20 Arg- oder/und Lys-Reste enthält. Ferner ist bevorzugt, wenn die Polykation-Komponente eine Serie von mindestens 3 aufeinanderfolgenden Arg- oder/und Lys-Resten, besonders bevorzugt von mindestens 4 aufeinanderfolgenden Arg- oder/und Lys-Resten enthält.

Das für das erfindungsgemäße Verfahren verwendete Protein enthält eine Komponente, die eine lineare genetische Fusion aus der Cell Homing Faktor- und der Polykation-Komponente darstellt. Dabei ist es bevorzugt, wenn die Polykation-Komponente des Proteins an das N- oder/und C-terminale Ende der Cell Homing Faktor-Komponente angefügt ist. Es ist jedoch auch möglich, die Polykation-Komponente nicht an den Enden der Cell Homing Faktor-Komponente, sondern auch innerhalb ihres Bereichs einzuführen. Diese Ausführungsform des erfindungsgemäßen Verfahrens kann insbesondere bei Proteinen angewandt werden, bei denen bekannt ist, daß in der Quartärstruktur Domänen, die nach außen ragen, vorliegen und die durch Zwischenschaltung einer Polykationkette einerseits ihre Funktionalität beibehalten würden, andererseits aber auch die Fähigkeit zur Bindung einer Nukleinsäure gewinnen würden. Ferner ist es natürlich auch möglich, wenn das Protein mehrere Polykation-Komponenten, z.B. am N- und am C-terminalen Ende der Cell Homing Faktor-Komponente enthält.

Die für das erfindungsgemäße Verfahren verwendbaren Proteine sind durch rekombinante DNA-Techniken auf einfache Weise erhältlich. So kann an das Ende, an die Enden oder/und innerhalb des für einen Cell Homing Faktor codierenden Gens durch Ligationsreaktionen mit einem geeigneten DNA-Adapter oder durch in vitro Mutagenese die für die Polykationkette codierenden DNA-Fragmente angefügt werden. Derartige Methoden der DNA-Manipulation sind dem Fachmann auf dem Gebiet der Molekularbiologie geläufig und brauchen daher an dieser Stelle nicht detailliert erläutert werden. In den Beispielen wird die für die Polykationketten codierende genetische Information durch Ligation chemisch synthetisierter DNA-Adaptermoleküle an die Cell Homing Faktor-Gene angefügt. Für den Fachmann ist jedoch klar, daß auch andere Methoden zu denselben Fusionsgenen führen können.

Ein weiterer Gegenstand der vorliegenden Erfindung ist ein Verfahren zur Einschleusung von Nukleinsäuren in Zielzellen, welches dadurch gekennzeichnet ist, daß man einen Komplex aus einem oben genannten Protein und einer Nukleinsäure mit einer Zielzelle unter geeigneten Bedingungen in Kontakt bringt, die zur Internalisierung des Komplexes bzw. der Nukleinsäure führen. Als Zielzellen werden vorzugsweise eukaryontische Zellen verwendet. Die Aufnahme von Nukleinsäuren durch die Zielzellen kann auf einfache Weise getestet werden, indem man radioaktiv markierte Nukleinsäuren zur Einschleusung in die Zielzellen verwendet. Unter geeigneten Bedingungen, die zur Internalisierung des Komplexes bzw. der Nukleinsäure führen, sind solche Bedingungen zu verstehen, bei denen die Zielzelle lebensfähig und zur Internalisierung des Cell Homing Faktors in der Lage ist.

Schließlich betrifft die vorliegende Erfindung noch die Verwendung eines Proteins, das eine lineare genetische Fusion einer zellspezifischen Cell Homing Faktor-Komponente mit mindestens einer Polykation-Komponente darstellt, zur Herstellung von Protein-Nukleinsäure-Komplexen, die unter physikalischen Bedingungen stabil sind, wobei eine Polykation-Komponente des Proteins eine Peptidsequenz ist, die mindestens 3 Aminosäuren, ausgewählt aus der Gruppe, bestehend aus Arginin und Lysin, enthält.

Die Erfindung soll weiterhin durch die folgenden Beispiele sowie den SEQ ID NO:1 - 9 erläutert werden.

Die in der vorliegenden Anmeldung genannten Plasmide und Mikroorganismen wurden bei der Deutschen Sammlung von Mikroorganismen und Zellkulturen GmbH (DSM), Mascheroder Weg 1B, D-3300 Braunschweig, unter den folgenden Hinterlegungsnummern hinterlegt:

| | |
|---|---|
| pKK177-3-G-CSF-Bg | DSM 5867 |
| PACYC 177 | DSM 3693 p |
| E. coli C 600 | DSM 5447 |
| E. coli C 600+ | DSM 5443 |
| pUBS 520 in E. coli C.600+ | DSM 6786 |
| pNGF1/pUBS 520 in E.coli C 600+ | DSM 6785 |

4

Beispiel 1

Testsystem auf DNA-Bindung

Der Test zur Untersuchung, ob ein Protein DNA-bindende Eigenschaft besitzt, sieht wie folgt aus:
Es wird zunächst eine Verdünnungsreihe der zu untersuchenden Proteinlösung hergestellt. Als Verdünnungspuffer wird 25 mmol/l NaCl verwendet. Es werden Proteinlösungen mit Proteinkonzentrationen zwischen 1 mg/ml und 0,01 mg/ml hergestellt.

Als Test-DNA wird Lambda-DNA, welche mit HindIII restringiert ist, verwendet. Die DNA-Lösung wird nach der Restriktion mit 25 mmol/l NaCl auf eine Konzentration von 50 ng/$\mu$l verdünnt.

Zur Durchführung der Tests werden 6 $\mu$l DNA-Lösung vorsichtig mit jeweils 6 $\mu$l Protein-Lösung gemischt und für 30 Minuten bei 25°C inkubiert.

Polylysin (Positiv-Kontrolle) und Polyglutamat (Negativ-Kontrolle) in unterschiedlichen Konzentrationen (0,5 mg/ml, 0,1 mg/ml, 0,01 mg/ml) werden auf die o.b. Weise mit Lambda-DNA gemischt und inkubiert.

Die Analyse auf DNA-Bindung findet durch Auftrennung der Komplexe in der Agarose-Gelelektrophorese statt: bei Vorhandensein eines stabilen Protein-DNA-Komplexes ändert sich das Wanderungsverhalten der Banden der restringierten DNA. Der Komplex aus DNA und Protein zeigt gegenüber der nicht-komplexierten DNA ein verzögertes Laufverhalten.

Beispiel 2

Herstellung von Proteinen mit G-CSF als "Cell Homing Faktor"-Komponente

1. Konstruktionen der Vektoren

1.1 N-terminale Fusionen

Das G-CSF-Gen im Vector pKK177-3-G-CSF-Bg (DSM 5867) wird unter Zuhilfenahme synthetischer DNA-Adapter so modifiziert, daß G-CSF-Derivate entstehen, welche an ihrem N-Terminus folgende zusätzliche Aminosäuren enthalten:

```
G—CSF (6+): MetLysAlaLysArgPheLysLysHisProArgProPro—G—CSF          (1.)

G—CSF (4+):              ArgPheLysLysHisProArgProPro—G—CSF          (2.)

G—CSF (5+): MetLysAlaLysAlaAlaArgPheLysHisProArgProPro—G—CSF       (3.)
```

(1.) = SEQ ID NO:1
(2.) = SEQ ID NO:2
(3.) = SEQ ID NO:3

Zur Konstruktion der Konjugate wird der Vektor pKK177-3-G-CSF-Bg (DSM 5867) mit EcoRI partial und ApaI verdaut und das Oligonukleotid

```
EcoRI                                                      Apa I
    AAATTCGGAGGAAAAATTA|ATG..........|ACACCACTGGGCC
                       |             |
                       |Met..........|(G—CSF—Sequenz ohne ATG)
```

in das entstandene linearisierte Vektorfragment (ca. 3450 bp) insertiert.

$$\text{AAATTCGGAGGAAAAATTA} \quad = \text{SEQ ID NO:4}$$

$$\text{ACACCACTGGGCC} \quad = \text{SEQ ID NO:5}$$

Die in die Lücke jeweils eingesetzte DNA-Sequenz entspricht dem genetischen Code für die oben genannnten Aminosäuren, d.h. beispielsweise für Konstrukt G-CSF (6 +) wurde ein Oligonukleotid mit dem genetischen Code für die Aminosäuresequenz LysAlaLysArgPheLysLysHisProArgProPro (≙ SEQ ID NO:1, ohne Met) eingesetzt. Die durch Ligation der Oligonukleotide in den geschnittenen Vektor resultierenden Plasmide werden in E.coli HB101 transformiert. Die G-CSF(4 +)-Variante kann weiterhin auch erzeugt werden, wenn das Konstrukt zur Expression von G-CSF (6 +) in E.coli-Zellen transformiert wird, die eine OmpT-Protease (Spaltung Lys| Arg) exprimieren (z. B. E.coli C600 (DSM 5447)). Um eine bessere Regulierbarkeit des tacPromotors zu gewährleisten, wurden die Zellen zusätzlich mit einem zu pBP010 (vgl. EP-A 0 464 832) kompatiblen Plasmid transformiert, das das lacI$^q$-Gen enthält. Das lacI$^q$-Gen ist dem Fachmann schon lange bekannt und leicht erhältlich. Als kompatible Plasmide zu pBP010 sind z.B. pACYC 177 (DSM 3693P), in welches das lacI$^q$-Gen insertiert ist, oder davon abgeleitete Plasmide geeignet (vgl. z.B. Gene 85 (1989), 109-114 und EP-A 0 373 365). Die resultierenden, mit beiden Plasmiden positiv transformierten

Klone werden auf Kanamycin (50 $\mu$g/ml)/ Ampicillin (50 $\mu$g/ml) selektiert und über Restriktionsanalyse identifiziert. Beim Schnitt mit EcoRI und EcoRV ergeben sich Fragmente der Längen von ca. 3.15 kb und ca. 0.3 kb (mit den jeweiligen Konstrukten).

## 1.2. C-terminale Fusion

Zur Klonierung der C-terminalen Fusion wurde das Expressionsplasmid pKK177-3 CG10 herangezogen. Dieses Plasmid kann durch Mutagenese aus dem Plasmid pKK177-3-G-CSF-Bg (DSM 5867) hergestellt werden. Dazu wurden mittels gerichteter Mutagenese die Codons der ersten drei Aminosäuren des maturen G-CSF-Gens im Plasmid pKK177-3 Bg so abgeändert, daß die Codons jeweils in der dritten Nukleotidposition ein A anstelle des ursprünglichen Nukleotids enthalten. Die Mutagenese wurde nach der Methode von Moringa et al., Biotechnology 21 (1984) 634 durchgeführt. Für die Mutagenese wurde folgendes Oligonukleotid eingesetzt:

5'-ATTAATGACACCACTAGGCCCTGCC-3' (SEQ ID NO:6)

Die erhaltenen positiv mutagenisierten Klone wurden mittels Sequenzanalyse verifiziert.

An das G-CSF-Gen, das im Expressionsplasmid pKK177-3 CG10 vorliegt, wurde ein chemisch synthetisierter doppelsträngiger DNA-Adapter angehängt, der wie folgt aussieht und für das C-terminale Ende von G-CSF zuzüglich acht Argininreste und einen Prolinrest kodiert:

$$5'-\text{CTAGCCATCTGCAGAGCTTCCTGGAGGTGTCGTACCGCGTTCTACG}-$$

$$3'-\text{GGTAGACGTCTCGAAGGACCTCCACAGCATGGCGCAAGATGC}-$$

$$-\text{CCACCTTGCGCAGCCCCGTCGTCGCCGTCGTCGCCGTCGTCCGTGATAA}-3'$$

$$-\text{GGTGGAACGCGTCGGGGCAGCAGCGGCAGCAGCGGCAGCAGGCACTATTTCGA}-5'$$

(SEQ ID NO:7 zeigt die Sequenz des chemisch synthetisierten doppelsträngigen DNA-Adapters, wobei der 5'3'-Strang aus den Basen 1 - 95 besteht und der Gegenstrang aus den zu 5 - 99 komplementären Basen.)

Dazu wurde das Plasmid pKK177-3 CG10 mit den beiden Restriktionsendonukleasen NheI und HindIII gespalten. Der Restriktionsansatz wurde gelelektrophoretisch aufgetrennt und das größere der beiden entstandenen Fragmente präparativ gewonnen. Der G-CSF enthaltende Vektor wurde mit dem obigen DNA-Adapter ligiert. Dazu wurden 1 $\mu$g des Vektorfragments und 1 $\mu$g des Adapters in 20 $\mu$l Ligationspuffer (10 x Puffer entspricht 0,5 mol/l Tris HCl pH 7,6, 100 mmol/l Magnesiumchlorid, 100 mmol/l Dithiothreitol und 500 $\mu$g/ml Rinderserumalbumin) aufgenommen. Die Ligation erfolgte nach Zugabe von 1 U T4-Ligase über Nacht bei 16 °C. Der Ligationsansatz wurde in E.coli (C600+, pUBS 520) transformiert. Die Richtigkeit des Klons wurde durch Sequenzierung überprüft. Das von diesem Klon exprimierte G-CSF-Derivat wurde G-CSF (8+) genannt.

2. Reinigung der G-CSF-Derivate

2.1. Fermentation:

Die gemäß 1.1 bzw. 1.2 positiv identifizierten Klone werden in 5 ml-Kultur in LB-Medium mit Kanamycin und Ampicillin (Konzentrationen jeweils 50 μg/ml) bis zu einer optischen Dichte bei 550 nm (OD) von 0,5 angezogen, mit 5 mmol/l IPTG induziert und 3 Stunden bei 37°C inkubiert. Die 10 OD entsprechende Menge von dieser induzierten Kultur werden geerntet und daraus ein Gesamtzellextrakt hergestellt. Der Gesamtzellextrakt wird auf einem SDS-Page Gel analysiert.

Wenn daraus ersichtlich ist, daß das gewünschte Protein exprimiert wird, wird die Kultur im 1 Liter-Maßstab wiederholt, die Zellen geerntet und eine Präparation von Inclusion Bodies (IB) durchgeführt.

2.2. IB-Präparation:

Die Zellen werden durch Zentrifugation geerntet, in 100 ml Tris-Magnesiumpuffer (10 mmol/l Tris, pH 8,0, 1 mmol/l MgCl$_2$) aufgenommen und mit Lysozym (0,3 mg/ml) aufgeschlossen.

Es wird 15 Minuten bei 37°C inkubiert und ein French-Press-Durchgang durchgeführt (1200 psi). Anschließend erfolgt ein DNAse-Verdau (2 mg DNAse I) bei 30 Minuten und 37°C.

Es werden 20 ml 0,5 mol/l NaCl, 20 mmol/l EDTA, pH 8,0 und 3 ml 20 % Triton® x 100 zugegeben und 10 Minuten bei 25°C inkubiert.

Die Suspension wird 10 Minuten bei 15.000 Upm und 4°C zentrifugiert. Das Pellet wird in 30 ml 50 mmol/l Tris, pH 8,0, 50 mmol/l EDTA und 0,5 % Triton® X 100 aufgenommen und mit Ultraschall behandelt. Es wird wieder zentrifugiert, resuspendiert und mit Ultraschall behandelt. Diese Prozedur wird noch zweimal wiederholt. Anschließend wird zentrifugiert und die so erhaltenen Pellets als IBs zur Solubilisierung/Renaturierung eingesetzt.

2.3. Solubilisierung/Renaturierung:

2.3.1 Solubilisierung

Solubilisierungspuffer:
6 mol/l Guanidin-Hydrochlorid
0,1 mol/l Tris-Puffer, pH 8,0
1 mmol/l EDTA
100 mmol/l DTE (Dithioerythritol)
Dialysepuffer (1):
6 mol/l Guanidin-Hydrochlorid
3 mmol/l EDTA bei pH 3,0
1 g Inclusion Bodies werden zu 30 ml Solubilisierungspuffer gegeben, 5 Minuten mit Ultraschall homogenisiert und eine Stunde bei Raumtemperatur inkubiert. Es wird HCl zugegeben, bis der pH-Wert 3,0 erreicht ist. Anschließend wird unlösliches Material abzentrifugiert.

Es wird gegen Dialysepuffer (1) dialysiert, bis DTE vollständig (≦ 1 mmol/l DTE) entfernt ist.

2.3.2 Pulsreaktivierung:

Renaturierungspuffer:
0,5 mol/l Arginin-Hydrochlorid
0,1 mol/l Tris-Puffer, pH 8,0
0,5 mmol/l GSH
0,5 mmol/l GSSG
1 mmol/l EDTA
Dialysepuffer (2):
10 mmol/l Tris-Puffer, pH 8,0
1 mmol/l EDTA

EP 0 544 292 A2

Die Pulsreaktivierung erfolgt wie in EP-A 0 241 022 beschrieben. Es wird eine Vorrichtung gemäß Fig. 5 von EP-A 0 241 022 verwendet.

Dazu wird in Zeitintervallen von 30 Minuten Protein in das Reaktionsvolumen (100 ml Renaturierungspuffer) gegeben, so daß die Proteinkonzentration im Reaktionsvolumen pro Puls um 50 $\mu$g/ml steigt. Insgesamt wird 20 mal gepulst (Endkonzentration ca. 1 mg/ml Reaktionsvolumen).

Nach der Pulsreaktivierung werden aus dem Reaktionsvolumen Trübungen abzentrifugiert und das gesamte Reaktionsvolumen gegen Dialysepuffer (2), bis zur Entfernung von Arginin ($\leq$ 50 mmol/l) dialysiert.

### 3. Bestimmung der Aktivität der G-CSF-Derivate

Die Aktivität von G-CSF wird mit der murinen Leukämie-Zellinie NFS60, die vollkommen G-CSF-abhängig ist, wie in Biochem.J. 253 (1988) 213-218, Exp.Hematol. 17 (1989) 116-119, Proc. Natl.Acad.Sci. USA 83 (1986) 5010 beschrieben, getestet. Damit die Faktorabhängigkeit der Zellen erhalten bleibt, enthält das Medium (RPMI Medium, Boehringer Mannheim GmbH, Best.Nr. 2099445 mit 10 % fötalem Kälberserum) der Erhaltungskultur permanent 1000 U/ml G-CSF.

Gemessen wird bei diesem Test direkt die G-CSF-stimulierende Proliferation der NFS60-Zellen durch den Einbau von $^3$H-Thymidin. Die Durchführung des Tests erfolgt folgendermaßen:

NFS60-Zellen, die sich in der exponentiellen Wachstumsphase befinden (Zelldichte maximal $1\times10^5$ Zellen/ml) werden in Mikrotiterplatten überführt ($1\times10^4$ Zellen/Loch) und mit einer abnehmenden G-CSF-Konzentration kultiviert. Die maximale Dosis von G-CSF in Loch 1 entspricht der Konzentration in der Erhaltungskultur (1000 U/ml, spezifische Aktivität $1\times10^8$ U/mg Protein). Die Verdünnung erfolgt in Zehnerschritten.

Nach etwa 24 Stunden Inkubation erfolgt die Zugabe von $^3$H-Thymidin (0,1 $\mu$Ci/Loch). Daraufhin werden die Zellen noch weitere 16 Stunden inkubiert.

Für die Auswertung der Tests werden die Zellen in der Mikrotiterplatte eingefroren, so daß sie lysiert werden. Das Zell-Lysat wird auf einen Glasfaserfilter gesaugt, gespült, getrocknet und im Szintillationszähler gemessen. Der Einbau von $^3$H-Thymidin ist proportional zur G-CSF induzierten Proliferation der NFS60-Zellen.

### 4. Bestimmung der DNA-Bindung

Zur Bestimmung der DNA-Bindung wurde der in Beispiel 1 beschriebene Test herangezogen. Das Ergebnis der Studie ist in Tabelle 1 wiedergegeben:

Tabelle 1

| Protein/Peptid | Ausgangskonz. (mg/ml) | Bandenänderung |
|---|---|---|
| G-CSF (8 +) | 0.5 | + + |
| " | 0.1 | + + |
| " | 0.05 | + |
| " | 0.01 | - |
| G-CSF (6 +) | 1.0 | + + + |
| " | 0.5 | + + |
| " | 0.1 | - |
| G-CSF (5 +) | 1.0 | + + |
| " | 0.5 | + + |
| " | 0.1 | - |
| G-CSF (4 +) | 1.0 | + |
| " | 0.5 | + |
| " | 0.1 | - |
| G-CSF (wt) | 1.0 | - |
| " | 0.5 | - |
| " | 0.1 | - |
| Poly-Lys | 0.1 | + + + |
| " | 0.03 | + + + |
| " | 0.01 | + |
| Poly-Glu | 0.1 | - |
| " | 0.03 | - |
| " | 0.01 | - |

## 5. Test auf Bindung des Komplexes an Zellen

Um zu testen, ob der Komplex aus Nukleinsäure und Fusionsprotein an die entsprechenden Rezeptoren auf der Oberfläche der Rezeptor exprimierenden Zellen bindet, wurde folgender Assay durchgeführt:

### 5.1 Komplex-Herstellung

Pro zehn Zelltests wurden 10 $\mu$g HindIII-restringierte Lambda-DNA mit $^{35}$S-dATP mittels Random-Priming (Boehringer Mannheim GmbH, Kat.Nummer 1004760) markiert. Zur Komplexbildung wurde die markierte DNA mit 100 $\mu$g Fusionsprotein bei 25°C gemischt. Anschließend erfolgte eine Inkubation für 30 Minuten bei derselben Temperatur.

### 5.2 Zelltest

Pro Test wurden 1 x 100.000 NFS60-Zellen in einem Volumen von 0.2 ml RPMI-Medium (mit 10 % FKS) mit 1 - 10 $\mu$g Fusionsprotein, komplexiert mit der radioaktiv markierten Nukleinsäure, versetzt. Dieser Ansatz wurde für 2, 4 bzw. 22 Stunden bei 37°C/5 % $CO_2$ inkubiert. Zur Auswertung wurden die Zellen 15 Minuten bei 2000 rpm abzentrifugiert, der Überstand wurde dekantiert und die Zellen mit 1 ml PBS (10 mmol/l Kaliumphosphatpuffer pH 7,5, 140 mmol/l NaCl) aufgenommen. Nach erneuter Zentrifugation wurde das Zellpellet in 100 $\mu$l PBS gelöst und eine Stunde bei -20°C tiefgefroren, um die Zellen aufzubrechen. Anschließend wurden die Zellen im Szintillationszähler vermessen.

Folgende Kontrollen wurden in Parallelansätzen durchgeführt:
- Inkubation der Zellen mit markierter DNA, ohne Zusatz von Protein.
- Inkubation der Zellen mit nativem G-CSF (wt), das wie oben beschrieben, mit der markierten DNA inkubiert worden war.

Es wurde der oben beschriebene Zelltest durchgeführt. Die Ergebnisse der Untersuchung sind in Tabelle 2 zusammengefaßt.

Tabelle 2

| Protein/Nukl.-Komplex | Zeit (Std.) | cpm[1] |
|---|---|---|
| G-CSF (6 + )/DNA | 2 | 37785 |
| " | 4 | 15765 |
| " | 22 | 135645 |
| G-CSF (wt)/DNA | 2 | 7455 |
| " | 4 | 3465 |
| " | 22 | 8580 |
| --/ DNA | 2 | 1305 |
| " | 4 | 2925 |
| " | 22 | 2325 |
| Tabelle 2 zeigt eindeutig, daß nur bei Anwesenheit von modifiziertem G-CSF(6 + ) ein Einbau von Radioaktivität, d.h. von Nukleinsäure in die Zellen gemessen wird. | | |

[1] counts per minute

Beispiel 3

Herstellung von Proteinen mit NGF als "Cell Homing Faktor"-Komponente

1. Konstruktion von Vektoren

Zur Herstellung des NGF-Derivats, das an seinem N-Terminus einen Fusionsanteil mit überwiegend positiven Aminosäuren enthält, wurde ein Expressionsplasmid wie folgt hergestellt: Es wurden dazu drei Ansätze ligiert. Ansatz A stellt das etwa 4.6 kb große EcoRI/BamHI-Fragment des Plasmids pBTac1 (Boehringer Mannheim GmbH, Kat.Nr. 1081365) dar. Ansatz B wurde durch Restriktion des Plasmids pUC18-NGF (British Biotechnology, Code BBG26) mit den Enzymen BspMI und BamHI hergestellt. Es wurde ein 360 bp großes BspMI/BamHI-Fragment präparativ gewonnen, welches die für NGF kodierende Region enthält. Ansatz C stellt folgenden, synthetisch hergestellten Adapter dar:

```
5'-AATTCTTATGTCTTACCAAAGAACTCACCGTAGCAAGCGC-3'
   3'-GAATACAGAATGGTTTCTTGAGTGGCATCGTTCGCGAGTA-5'
      MetSerTyrGlnArgThrHisArgSerLysArg
```

(SEQ ID NO:8 zeigt die Sequenz des synthetisierten Adapters, wobei der 5'-3'-Strang aus den Basen 1 - 40 besteht und der Gegenstrang aus den zu 5 - 44 komplementären Basen. Die Aminosäuresequenz wird nochmals gesondert in SEQ ID NO:9 dargestellt.)

Die drei Ansätze wurden nach bekannten Methoden ligiert, wobei Ansatz A und B jeweils in einer Konzentration von 500 ng, Ansatz C in einer Konzentration von 1 µg eingesetzt wurden. Der Ligationsansatz wurde in E.coli C600 + (DSM 5443), welche das Plasmid pUBS520 (Brinkmann et al., 1989) enthält (DSM 6786), transformiert. Durch Selektion auf Nährmedien, welche Ampicillin (50 µg/ml) und Kanamycin (50 µg/ml) enthielten, konnten die gewünschten Klone erhalten werden. Das erhaltene Plasmid pNGF1 unterscheidet sich vom Ausgangsvektor durch das Auftreten einer zusätzlichen ScaI-Schnittstelle. Das Plasmid pNGF1 ist als Plasmidgemisch mit pUBS 520 in E. coli C 600+ als Wirtsstamm unter DSM 6785 hinterlegt.

2. Reinigung

2.1. Präparation von Inclusion bodies ( = IBs)

Zur Präparation der rec. NGF-enthaltenden IBs wurde der in Beispiel 3.1 beschriebene Expressionsstamm E.coli C600+ mit den Plasmiden pNGF1 und pUBS 520 in einem 10 l-Fermenter für 8 Stunden fermentiert. Die Induktion der Expression des NGF-Derivats erfolgte durch die Zugabe von IPTG in der logarithmischen Wachstumsphase ca. 4 Stunden nach Fermentationsbeginn.

690 g Biomasse wurde nach 8 Stunden Fermentation mittels Zentrifugation geerntet. Die Biomasse wurde in 3,5 l 0,1 mol/l Tris/HCl pH 7 suspendiert und nach Zugabe von 0,3 g Lysozym 20 Minuten bei 0°C inkubiert. Der vollständige Zellaufschluß wurde anschließend mittels Hochdruckdispension mit 600 bar durchgeführt. Zur Aufschlußlösung wurde DNase ad 0,1 mg/ml und $MgSO_4$ ad 2 mmol/l zugegeben und die Lösung 30 Minuten bei 20°C inkubiert. Nach der DNase-Behandlung wurde die Lösung mit demselben Volumen 0,1 mol/l Tris/HCl, 6 % Triton® X 100, 1,5 mol/l NaCl, 60 mmol/l EDTA, pH 7,0 verdünnt und 20 Minuten im Eisbad inkubiert. Unlösliche Bestandteile (IBs) wurden anschließend durch Zentrifugation abgetrennt. Der Niederschlag wurde in 1 l 0,5 mol/l Guanidiniumchlorid (GdmCl), 20 mmol/l EDTA, pH 4,5 suspendiert. Nach 20 Minuten Inkubation bei 20°C wurden die IBs durch erneute Zentrifugation geerntet. Die folgende Resuspension des Niederschlags erfolgte in 1,5 l 0,1 mol/l Tris/HCl, 20 mmol/l EDTA, pH 6,5. Nach 30 Minuten Inkubation bei 20°C wurden die IBs durch eine weitere Zentrifugation im Niederschlag erhalten.

## 2.2. Pulsrenaturierung von rec NGF

20 g IB-Material wurde in 400 ml 6 mol/l GdmCl, 0,1 mol/l Tris/HCl, 0,1 mol/l DTE, 2 mmol/l EDTA pH 8,5 gelöst und 1 h bei 20°C inkubiert. Anschließend wurde der pH-Wert der Lösung auf pH 3 mit 25 % HCl eingestellt und gegen 3 x 10 l 6 mol/l GdmCl, 2 mmol/l EDTA, pH 3 bei 4°C dialysiert. Je 100 ml Dialysat wurden im Abstand von 12-16 h innerhalb von 30 Minuten zu 20 l 0,7 mol/l Arginin, 2 mol/l Harnstoff, 0,1 mol/l Tris, 2 mmol/l EDTA, 5 mmol/l Cysteamin, 1 mmol/l Cystamin, pH 9,1, 4°C zugepumpt.

## 2.3. Reinigung von biologisch aktivem rec. NGF aus der Renaturierungslösung

Zu der Renaturierungslösung wurde festes Ammoniumsulfat portionsweise bis zu einer Sättigung von 20 % zugegeben und 1 h auf Eis gerührt. Der pH-Wert der Lösung wurde anschließend mit 25 %iger HCl auf pH 3 eingestellt. Nach 30 Minuten Inkubation wurde mit 7 mol/l NaOH auf pH 8 titriert. Trübungen wurden entweder durch Zentrifugation (1 h mit 20 000 g bei 4°C) oder Filtration durch eine Membran mit geringer Proteinadsorption (z. B. Evaflux A 4 Hohlfaserpatrone der Fa. Kawasumi Laboratories, Japan) entfernt. Die klare NGF-enthaltende Lösung wurde dann auf eine TSK-Butyl-Säule (Fa. Pharmacia) aufgetragen, die vorher mit 50 mmol/l Tris/HCl, 1 mmol/l EDTA, 20 % Sättigung an $(NH_4)_2SO_4$, pH 7,5 äquilibriert wurde. Nach Beendigung des Probenauftrags wurde mit 50 mmol/l Tris/HCl, 1 mmol/l EDTA pH 7,5 gespült. Die Elution erfolgte mit 50 % Ethylenglykol in 50 mmol/l Na-Acetat, 1 mmol/l EDTA, pH 4,5. Eluierende Fraktion wurden auf den Gehalt an NGF getestet. NGF enthaltende Fraktionen wurden vereint und auf eine Sepharcryl S200-Säule (Fa. Pharmacia, Schweden) aufgetragen, die mit 0,5 mol/l NaCl, 50 mmol/l Na-Phosphat, 10 % Glycerin, 1 mmol/l EDTA, pH 6,0 äquilibriert war. Die Säule wurde mit demselben Puffer eluiert.

NGF enthaltende Fraktionen im Eluat wurden mit $H_2O$ auf eine Leitfähigkeit von 15 mS verdünnt und auf eine mit 20 mmol/l Na-Phosphat, 1 mmol/l EDTA, pH 6,0 äquilibrierte S-Sepharose-Säule (Fa. Pharmacia) aufgetragen. Anschließend wurde mit dem Äquilibrierungspuffer gespült. Die Elution erfolgte mit einem Gradienten bis 1,2 mol/l NaCl in obigen Puffer. NGF enthaltende Fraktionen wurden vereinigt und, sofern für weitere Analysen erforderlich, durch Dialyse umgepuffert.

## 3. Bestimmung der Aktivität des NGF-Fusionsproteins

Für die Bestimmung der Aktivität des NGF-Fusionsproteins werden zunächst Dorsalwurzelganglien aus bebrüteten Hühnereiern isoliert. Dazu werden acht Tage bebrütete Hühnereier zur Desinfektion mit 70 %-igem Ethanol abgesprüht, mit einer stumpfen Pinzette die Spitze aufgeschlagen und Eierschale sowie Eihaut entfernt. Mit einer gebogenen Pinzette wird sodann der Embryo herausgefischt, in eine Petrischale überführt und der Kopf abgetrennt. Der Embryo wird dann eine zweite Petrischale überführt, auf den Rücken gelegt und mit einer stumpfen Pinzette am Hals fixiert. Mit einer kleinen Präparierschere wird vom Schwanz her bis zum Brustraum ein Schnitt geführt, mit einer gebogenen Pinzette die Eingeweide entfernt, bis das Rückenmark freiliegt. Der Embryo wird anschließend mit sterilem PBS abgespült, bevor mit einer Uhrmacherpinzette die sympathischen Grenzstränge entfernt werden, wodurch die Dorsalwurzelganglien sichtbar werden. Mit der Uhrmacherpinzette wird dann in Richtung Schwanz am Rande des Rückenmarks entlanggefahren, so daß sich die Ganglien ablösen und abgeerntet werden können. Die möglichst vollständigen Ganglien werden in einer kleinen auf Eis stehenden Petrischale, die mit sterilem PBS gefüllt ist, gesammelt. Die präparierten Ganglien werden in ein 15 ml-Röhrchen überführt, steriles PBS ad 2 ml zugegeben und anschließend mit 80 $\mu$l 2,5 %-iger Trypsinlösung für 30 Minuten bei 37°C im Wasserbad inkubiert, wobei etwa alle fünf Minuten kurz aufgeschüttelt wird. Das Gangliensediment wird anschließend in ein Röhrchen mit 5 ml F14-Medium (Zusammensetzung s. unten) überführt und kurz aufgeschüttelt. Anschließend läßt man die Ganglien wieder absitzen. Dieser Waschvorgang wird zweimal wiederholt. Anschließend werden die Ganglien dissoziiert, indem man sie mit einer Pipette gut resuspendiert, die trübe Zellsuspension in eine T25-Kulturflasche überführt und restliche Zellen aus dem Röhrchen mit Medium nachspült. Die Kulturflaschen werden dann für zwei Stunden zum Präplattieren der Zellen in den Brutschrank gestellt (27°C, 50 % relative Luftfeuchtigkeit 7,5 % $CO_2$). Nach diesem Präplattieren, bei dem sich nicht neuronale Zellen auf dem Boden der Kulturflasche absetzen, werden die neuronalen Zellen in mit Ornithin und Laminin beschichtete 24er- bzw. 96er Mikrotiterplatten überführt.

Zur Beschichtung mit Poly-DL-Ornithin werden von einer Lösung mit 500 $\mu$g/ml Poly-DL-Ornithin (Hydrobromid, Molekulargewicht 3000 - 15000 von Sigma, Bestell-Nr. P 8638) in 0,15 mol/l Boratpuffer jeweils 300 $\mu$l/Vertiefung der Mikrotiterplatten gegeben und danach bei 4°C auf einem Schüttler inkubiert. Anschließend wird die Poly-DL-Ornithin-Lösung abgesaugt und zweimal mit sterilem Aqua bidest gewaschen. Zur Beschichtung mit Laminin wird dann von einer Lösung mit 3,4 $\mu$g/ml Laminin (Boehringer Mannheim GmbH, Katalog-Nr. 1243217) in PBS jeweils 300 $\mu$l/Vertiefung der Mikrotiterplatte gegeben und vier Stunden bei Raumtemperatur auf einem Schüttler inkubiert. Die endgültige Vorbereitung der Platten erfolgt vor Ende des Präplattierens. Dazu wird die Lamininlösung abgesaugt und sofort 800 $\mu$l Medium zugegeben. Anschließend werden 100 $\mu$l der zu prüfenden Probe (verdünnt in Medium) sowie nach Abschluß der zweistündigen Präplattierungsphase 100 $\mu$l der neuronalen Zellen hinzugegeben.

Als Maß für die biologische Aktivität wurde die Anzahl der Zellen mit ausgebildeten Dendriten quantifiziert. Als Referenz wurde eine Lösung bekannter Konzentration von 2,55 NGF aus Submaxillaris-Drüsen der Maus (Boehringer Mannheim GmbH, Katalog-Nr. 1179195) verwendet.

| F 14 Medium: (F 12 Medium, modifiziert, Boehringer Mannheim GmbH, Katalog-Nr. 210 161) | | |
|---|---|---|
| 1. | F 12 Medium (Pulver) | 10,91 g |
| 2. | L-Glutamin, 200 mmol/l (Boehringer Mannheim GmbH, Katalog-Nr. 210 277) | 10 ml |
| 3. | Natriumpyruvat, 100 mmol/l (Biochrom KG: L 0473) | 20 ml |
| 4. | nicht essentielle Aminosäuren (Boehringer Mannheim GmbH, Katalog-Nr. 210 293) | 20 ml |
| 5. | Pferdeserum (inaktiviert) | 100 ml |
| 6. | Streptomycin-Penicillin (Boehringer Mannheim GmbH, Katalog-Nr. 210 404) | 2 ml |
| 7. | 23,5 mmol/l $NaHCO_3$ (Merck 6329) | 1,97 g |
| 8. | 11 mmol/l Glucose (Merck 4074) | 2,18 g |
| 9. | 5 mmol/l Kaliumchlorid (Merck 4936) | 373 mg |
| 10. | 2 mmol/l $CaCl_2$ x 2 $H_2O$ (Merck 2382) | 294 mg |
| 11. | 0,85 mmol/l $MgCl_2$ x 6 $H_2O$ (Merck 5833) | 173 mg |
| 12. | 0,15 mmol/l $MgSO_4$ x 7 $H_2O$ (Merck 5886) | 37 mg |
| 13. | 0,085 mmol/l Ascorbinsäure (Sigma A 4034) | 17 mg |
| 14. | 0,5 $\mu$mol/l $ZnSO_4$ x $H_2O$ (Merck 8882) 9 mg/10 ml | 200 $\mu$l |

4. Test auf DNA-Bindung

Der Test auf DNA-Bindung wurde wie in Beispiel 1 beschrieben durchgeführt. Die Ergebnisse der Untersuchung sind in Tabelle 3 aufgelistet.

Tabelle 3

| Protein / Peptid | Ausgangskonz. (mg/ml) | Bandenänderung |
|---|---|---|
| NGF ( + ) | 1.0 | + |
| " | 0.5 | + |
| " | 0.1 | - |
| Poly-Lys | 0.5 | + + + |
| " | 0.1 | + + + |
| " | 0.01 | + |
| Poly-Glu | 0.5 | - |
| " | 0.1 | - |
| " | 0.01 | - |

## Patentansprüche

1. Verfahren zur Herstellung eines Komplexes für die Einschleusung von Nukleinsäuren in Zielzellen durch nichtkovalente Komplexbildung einer Nukleinsäure mit einem Protein, das eine für die Zielzelle spezifische "Cell Homing Faktor"-Komponente und mindestens eine Polykation-Komponente enthält, **dadurch gekennzeichnet,** daß man ein Protein, das eine lineare genetische Fusion aus der "Cell Homing Faktor"- und mindestens einer Polykation-Komponente enthält, mit der in die Zielzellen einzuschleusenden Nukleinsäure unter geeigneten Bedingungen in Kontakt bringt, so daß ein im wesentlichen auf ionischen Wechselwirkungen zwischen der Nukleinsäure und dem Polykation-Anteil des Proteins beruhender Komplex gebildet wird, wobei eine Polykation-Komponente des Proteins eine Peptidsequenz ist, die mindestens 3 Aminosäuren, ausgewählt aus Lysin und Arginin, enthält und eine unter physiologischen Bedingungen stabile Bindung mit Nukleinsäuren bilden kann.

2. Verfahren nach Anspruch 1, **dadurch gekennzeichnet,** daß man eine "Cell Homing Faktor"-Komponente aus der Gruppe, bestehend aus Wachstumsfaktoren, Hormonen, viralen Antigenen, Toxinen, Lipoproteinen und Integrinen, verwendet.

3. Verfahren nach Anspruch 2, **dadurch gekennzeichnet,** daß man als "Cell Homing Faktor"-Komponente einen Wachstumsfaktor, insbesondere G-CSF oder NGF, verwendet.

16

4.  Verfahren nach einem der vorhergehenden Ansprüche,
    **dadurch gekennzeichnet,**
    daß man ein Protein verwendet, das nach Inkubation bei 25 °C in 25 mmol/l NaCl, einem pH zwischen 7 und 8 und in Gegenwart einer Nukleinsäurekonzentration von 25 ng/µl bei einer Proteinkonzentration von 0,01 - 1 mg/ml einen Protein-Nukleinsäure-Komplex mit ausreichender Stabilität bildet, um bei anschließender Agarose-Gelelektrophorese eine Retardation der Nukleinsäurebanden zu bewirken.

5.  Verfahren nach einem der vorhergehenden Ansprüche,
    **dadurch gekennzeichnet,**
    daß die Polykation-Komponente des Proteins mindestens 3 Arg- oder/und Lys-Reste enthält und einen Anteil von Arg und Lys von mindestens 40 % aufweist.

6.  Verfahren nach Anspruch 5,
    **dadurch gekennzeichnet,**
    daß die Polykation-Komponente 4 bis 20 Arg- oder/und Lys-Reste enthält.

7.  Verfahren nach einem der Ansprüche 5 bis 6,
    **dadurch gekennzeichnet,**
    daß die Polykation-Komponente eine Serie von mindestens 4 aufeinanderfolgenden Arg- oder/und Lys-Resten enthält.

8.  Verfahren nach einem der vorhergehenden Ansprüche,
    **dadurch gekennzeichnet,**
    daß die Polykation-Komponente des Proteins an das N- oder/und C-terminale Ende der "Cell Homing Faktor"-Komponente angefügt ist.

9.  Verfahren nach einem der vorhergehenden Ansprüche,
    **dadurch gekennzeichnet,**
    daß die in die Zielzelle einzuschleusende Nukleinsäure eine einzel- oder doppelsträngige, unmodifizierte oder modifizierte Deoxyribonukleinsäure oder Ribonukleinsäure ist.

10. Verfahren zur Einschleusung von Nukleinsäuren in Zielzellen,
    **dadurch gekennzeichnet,**
    daß man einen nach einem der Ansprüche 1 bis 9 hergestellten Komplex mit einer Zielzelle unter geeigneten Bedingungen in Kontakt bringt, die zur Internalisierung des Komplexes bzw. der Nukleinsäure führen.

11. Verwendung eines Proteins, das eine lineare genetische Fusion einer zellspezifischen "Cell Homing Faktor"-Komponente mit mindestens einer Polykation-Komponente darstellt, zur Herstellung von Protein-Nukleinsäure-Komplexen, die unter physikalischen Bedingungen stabil sind, wobei eine Polykation-Komponente des Proteins eine Peptidsequenz ist, die mindestens 3 Aminosäuren, ausgewählt aus der Gruppe, bestehend aus Arginin und Lysin, enthält.

SEQUENZPROTOKOLL


(1) ALGEMEINE INFORMATION:

        (i) ANMELDER:
            (A) NAME: BOEHRINGER MANNHEIM GMBH
            (B) STRASSE: Sandhofer Strasse 116
            (C) ORT: 6800 Mannheim 31
            (E) LAND: Germany
            (F) POSTLEITZAHL: 6800
            (G) TELEPHON: 0621/759-3197
            (H) TELEFAX: 0621/759-4457

        (ii) ANMELDETITEL: Verfahren zur Einschleusung von
                Nukleinsaeuren in Zellen

        (iii) ANZAHL DER SEQUENZEN: 9

        (iv) COMPUTER-LESBARE FORM:
            (A) DATENTRÄGER: Floppy disk
            (B) COMPUTER: IBM PC compatible
            (C) BETRIEBSSYSTEM: PC-DOS/MS-DOS
            (D) SOFTWARE: PatentIn Release #1.0, Version #1.25 (EPA)

        (vi) FRÜHERE ANMELDEDATEN:
            (A) ANMELDENUMMER: DE P 41 39 001.6
            (B) ANMELDEDATUM: 27-NOV-1991


(2) INFORMATION ZU SEQ ID NO: 1:

        (i) SEQUENZ CHARAKTERISTIKA:
            (A) LÄNGE: 13 Aminosäuren
            (B) ART: Aminosäure
            (D) TOPOLOGIE: linear


        (xi) SEQUENZBESCHREIBUNG: SEQ ID NO: 1:

        Met Lys Ala Lys Arg Phe Lys Lys His Pro Arg Pro Pro
        1               5                   10

(2) INFORMATION ZU SEQ ID NO: 2:

        (i) SEQUENZ CHARAKTERISTIKA:
            (A) LÄNGE: 9 Aminosäuren
            (B) ART: Aminosäure
            (D) TOPOLOGIE: linear


        (xi) SEQUENZBESCHREIBUNG: SEQ ID NO: 2:

        Arg Phe Lys Lys His Pro Arg Pro Pro
        1               5

(2) INFORMATION ZU SEQ ID NO: 3:

        (i) SEQUENZ CHARAKTERISTIKA:
            (A) LÄNGE: 14 Aminosäuren
            (B) ART: Aminosäure
            (D) TOPOLOGIE: linear


14

(xi) SEQUENZBESCHREIBUNG: SEQ ID NO: 3:

Met Lys Ala Lys Ala Ala Arg Phe Lys His Pro Arg Pro Pro
1               5                   10

(2) INFORMATION ZU SEQ ID NO: 4:

    (i) SEQUENZ CHARAKTERISTIKA:
       (A) LÄNGE: 19 Basenpaare
       (B) ART: Nukleinsäure
       (C) STRANGFORM: Einzel
       (D) TOPOLOGIE: linear

(xi) SEQUENZBESCHREIBUNG: SEQ ID NO: 4:

AAATTCGGAG GAAAAATTA               19

(2) INFORMATION ZU SEQ ID NO: 5:

    (i) SEQUENZ CHARAKTERISTIKA:
       (A) LÄNGE: 13 Basenpaare
       (B) ART: Nukleinsäure
       (C) STRANGFORM: Einzel
       (D) TOPOLOGIE: linear

(xi) SEQUENZBESCHREIBUNG: SEQ ID NO: 5:

ACACCACTGG GCC                  13

(2) INFORMATION ZU SEQ ID NO: 6:

    (i) SEQUENZ CHARAKTERISTIKA:
       (A) LÄNGE: 25 Basenpaare
       (B) ART: Nukleinsäure
       (C) STRANGFORM: Einzel
       (D) TOPOLOGIE: linear

(xi) SEQUENZBESCHREIBUNG: SEQ ID NO: 6:

ATTAATGACA CCACTAGGCC CTGCC         25

(2) INFORMATION ZU SEQ ID NO: 7:

    (i) SEQUENZ CHARAKTERISTIKA:
       (A) LÄNGE: 99 Basenpaare
       (B) ART: Nukleinsäure
       (C) STRANGFORM: beides
       (D) TOPOLOGIE: linear

(xi) SEQUENZBESCHREIBUNG: SEQ ID NO: 7:

CTAGCCATCT GCAGAGCTTC CTGGAGGTGT CGTACCGCGT TCTACGCCAC CTTGCGCAGC    60

CCCGTCGTCG CCGTCGTCGC CGTCGTCCGT GATAAAGCT                    99

(2) INFORMATION ZU SEQ ID NO: 8:

      (i) SEQUENZ CHARAKTERISTIKA:
          (A) LÄNGE: 44 Basenpaare
          (B) ART: Nukleinsäure
          (C) STRANGFORM: beides
          (D) TOPOLOGIE: linear

      (ix) MERKMALE:
          (A) NAME/SCHLÜSSEL: CDS
          (B) LAGE: 8..40

      (xi) SEQUENZBESCHREIBUNG: SEQ ID NO: 8:

AATTCTT ATG TCT TAC CAA AGA ACT CAC CGT AGC AAG CGC TCAT                    44
       Met Ser Tyr Gln Arg Thr His Arg Ser Lys Arg
        1         5           10

(2) INFORMATION ZU SEQ ID NO: 9:

      (i) SEQUENZ CHARAKTERISTIKA:
          (A) LÄNGE: 11 Aminosäuren
          (B) ART: Aminosäure
          (D) TOPOLOGIE: linear

      (ii) ART DES MOLEKÜLS: Protein

      (xi) SEQUENZBESCHREIBUNG: SEQ ID NO: 9:

Met Ser Tyr Gln Arg Thr His Arg Ser Lys Arg
 1        5         10